# EUROPÄISCHE PATENTSCHRIFT

(11) **EP 1 660 015 B1**
(45) Veröffentlichungstag und Bekanntmachung des Hinweises auf die Patenterteilung: **08.10.2008**
(21) Anmeldenummer: 04786218.0
(22) Anmeldetag: 24.08.2004
(51) Int. Cl.: A61K 8/26, A61Q 11/00, A61Q 11/02

(54) **MUND-UND ZAHNPFLEGEMITTEL**
ORAL AND DENTAL CARE PRODUCT
SOLUTION DENTAIRE ET BUCCALE

(30) Priorität: 01.09.2003 DE 10340543
(43) Veröffentlichungstag der Anmeldung: 31.05.2006
(73) Patentinhaber: Henkel AG & Co. KGaA, 40589 Düsseldorf (DE); Sustech GmbH & Co. KG, 64287 Darmstadt (DE)
(72) Erfinder: BARTH, Adolf-Peter, 42781 Haan (DE); KROPF, Christian, 40724 Hilden (DE); POTH, Tilo, 69469 Weinheim (DE)
(86) Internationale Anmeldenummer: PCT/EP2004/009438
(87) Internationale Veröffentlichungsnummer: WO 2005/020878

(56) Entgegenhaltungen:
- EP-A- 0 786 245
- WO-A-01/01930
- WO-A-01/95864

## Beschreibung

Die Erfindung betrifft Mund- und Zahnpflegemittel mit einem Gehalt an Kompositmaterialien und Putzmitteln, die durch spezifische Auswahl der Putzmittel eine optimale Reinigung bei gleichzeitiger Remineralisation der Zahnoberfläche gewährleisten.

Es sind bereits Mund- und Zahnreinigungsmittel bekannt, die ein Kompositmaterial aus schwerlöslichen, nanopartikulären Calciumsalzen und Proteinkomponenten in der Kombination mit Putz- und Poliermitteln enthalten. Beispielsweise ist in der WO 01/01930 A1 eine Zahncreme auf der Basis von etwa 10% abrasiven Kieselsäuren und 5% eines Kompositmaterials vorgeschlagen worden. Die Wirkung des Kompositmaterials beruht auf der Biomineralisation von Knochen- und Zahnmaterial durch Verschließung von Dentalläsionen auf der Zahnoberfläche. Abrasive Stoffe hingegen sind essentielle Bestandteile einer jeden Zahncremerezeptur und sind verantwortlich für die Reinigung der Zähne und die Beseitigung von Zahnverunreinigungen und -belägen durch Abrasion.

Aus der Offenbarung der EP 786 245 A1 sind zahlreiche Mund- und Zahnpflegemittel bekannt, die die Kombination eines nanopartikulären Hydroxylapatits und eines Putzmittels betreffen. Der Hydroxylapatit ist verantwortlich für die Remineralisierung von Läsionen auf der Zahnoberfläche und für die Prävention von Karies. Als Putzmittel, die in hohen Konzentrationen eingesetzt werden, dienen Aluminiumhydroxide und Calciumphosphate.

In der Praxis hat sich die Anwendung von Zahncremerezepturen gemäß WO 01/01930 A1 und EP 786 245 A1 als problematisch erwiesen, denn die Wirkung des Abrasivmittels und der remineralisierenden Komponente laufen einander zuwider. So wurde beobachtet, dass insbesondere bei der kurzen Einwirkungszeit einer Zahncreme im Mundraum von etwa zwei bis drei Minuten der Depoteffekt der remineralisierenden Komponente durch die Putz- und Polierwirkungwirkung des Abrasionsmittels limitiert wurde. Offenbar wurde die deponierte remineralisierende Komponente durch die Abrasivstoffe beim Putzvorgang zu großen Teilen wieder entfernt.

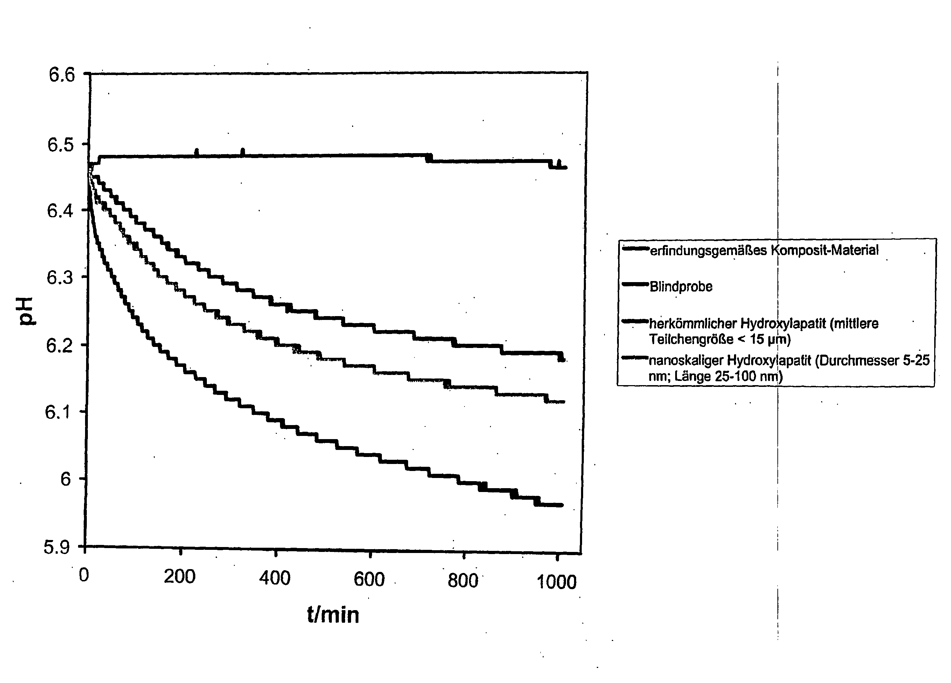

Deshalb kommt es darauf an, dass die remineralisierende Komponente sehr gute remineralisierende Eigenschaften besitzt, damit die remineralisierende Wirkung beim Putzvorgang noch zufriedenstellend ist. Der Vergleich verschiedener remineralisierender Komponenten wie herkömmlicher Hydroxylapatit, nanoskaliger Hydroxylapatit bzw. des erfindungsgemäßen Kompositmaterials in Abbildung 1 zeigt deutliche Unterschiede bezüglich ihrer remineralisierenden Eigenschaften.

Abbildung 1 zeigt den zeitlichen Verlauf des pH-Wertes, gemessen in einer 0,1%iger Dispersion des jeweiligen Materials in künstlichem Speichel bei 37°C. Für die vorliegende Untersuchung wurde künstlicher Speichel verwendet, der aus wässriger Lösung von 14mM Na⁺, 4,7mM PO₄³⁻, 21mM K⁺, 30mM Cl⁻, 1,8mM Ca²⁺ besteht und damit an Calciumphosphat übersättigt ist. Der pH-Wert wurde mittels einer pH-Elektrode (Inlab 410, Mettler Toledo; Meßgerät: Consört, Multi Parameter Analyzer C833) verfolgt.

Die zeitliche pH-Wert-Änderung resultiert aus der Bildung von Hydroxylapatit aus dem Speichel (also dem Remineralisierungseffekt), der der folgenden Gleichung genügt:

10 CaCl₂ + 6 Na₂HPO₄ + 2 H₂O → Ca₁₀(OH)₂(PO₄)₆ + 12 NaCl + 8 HCl

Bei dieser Reaktion wird Säure frei, der pH-Wert sinkt, und zwar umso stärker, je besser die remineralisierende Wirkung des getesteten Materials ist.

Die wesentlich steilere Abnahme des pH-Wertes im Falle des Kompositmaterials verglichen mit herkömmlichem Hydroxylpapatit oder nanoskaligem Hydroxylapatit zeigt die überlegenen remineralisierenden Eigenschaften des Kompositmaterials. Es ist damit hervorragend als remineralisierende Komponente in einer Zahncremeformulierung geeignet, in der dem Depoteffekt der remineralisierenden Komponente stets die Putz- und Polierwirkung eines Abrasionsmittels entgegensteht.

Neben der Remineralisierung sollen die erfindungsgemäßen Mund- und Zahnreinigungsmittel aber auch eine optimale Reinigungsleistung aufweisen, d.h. dass auf Putzmittel nicht verzichtet werden kann.

Sowohl die Menge, als auch die Art und Zusammensetzung des Putzmittels hat jedoch Einfluss auf die Remineralisierung, deshalb war die Aufgabe der vorliegenden Erfindung ein Mund- und Zahnreinigungsmittel herzustellen, das eine hohe Reinigungsleistung aufweist und gleichzeitig einen guten Depoteffekt der remineralisiernden Komponente zeigt.

Überraschenderweise wurde durch Kombination des Kompositmaterials mit einem Putzmittelgemisch, das einen Mindestgehalt an Aluminiumoxid umfasst, ein Mund- und Zahnpflegemittel hergestellt, das bei gleichbleibender Depotwirkung des Kompositmaterials über eine verbesserte Putzwirkung verfügt (im Vergleich zu den Beispielen der WO 01/01930). Dadurch wird eine besonders gründliche Reinigung der Zahnoberfläche bei gleichzeitiger Remineralisierung gewährleistet.

Darüber hinaus zeichnen sich die erfindungsgemäßen Zusammensetzungen durch einen Reparatureffekt aus. Unebenheiten und Beschädigungen des Zahnschmelzes, beispielsweise Kratzer auf dem Zahnschmelz durch mechanische Einwirkung, werden geglättet, indem sie durch Hydroxylapatit "aufgefüllt" werden. Neben der Reparatur beschädigter Zahnschmelzoberflächen führt dies zu einer ästhetisch ansprechenden Oberfläche. Zusätzlich wird schmerzempfindlichen Zähnen vorgebeugt, so dass die erfindungsgemäßen Zusammensetzungen eine sensitive Reinigung ermöglichen.

Gegenstand der vorliegenden Erfindung ist daher ein Mund- und Zahnpflegemittel, auf der Basis
a) eines Kompositmaterials, enthaltend
   - in Wasser schwerlösliche Calciumsalze in Form von nanopartikulären Primärteilchen mit einer Länge von 5 bis 150 nm und einem Querschnitt von 2 bis 50 nm und
   - Proteinkomponenten, ausgewählt aus Proteinen, Proteinhydrolysaten und Proteinhydrolysat-Derivaten, und
b) 10 bis 35 Gew.-% eines Putzkörpergemisches ,
wobei das Putzkörpergemisch 0,01 bis 5 Gew.-% Aluminiumoxid-Poliermittel enthält.

Unter Kompositmaterialien werden Verbundstoffe, welche die unter a) genannten Komponenten umfassen und mikroskopisch heterogene, makroskopisch aber homogen erscheinende Aggregate darstellen, und in welchen die Primärpartikel der Calciumsalze an das Gerüst der Proteinkomponente assoziiert vorliegen. Der Anteil der Proteinkomponente(n) in den Kompositmaterialien liegt zwischen 0,1 und 60 Gew.-%, bevorzugt aber zwischen 2 und 50 Gew.-%, insbesondere zwischen 20 und 50 Gew..-% bezogen auf das Gesamtgewicht der Kompositmaterlialien.

Unter Primärteilchen werden die Kristallite, d.h. die Einzelkristallite der genannten Calciumsalze verstanden. Als Teichendurchmesser soll hier der Durchmesser der Teilung in Richtung ihrer größten Längenausdehnung verstanden werden. Unter dem mitteleren Teilchendurchmesser ist ein über die Gesamtmenge des Komposits gemittelter Wert zu verstehen. Die Bestimmung der Teilchendurchmesser kann durch dem Fachmann geläufige Methoden bestimmt werden, beispielsweise mittels Scherrer-Analyse aus röntgendiffraktometrischen Untersuchungen.

Vorzugsweise liegt der mittlere Teilchendurchmesser der nanopartikulären Primärteilchen im Bereich von 5 bis 150 nm, und besonders bevorzugt liegen sie als stäbchenförmige Partikel vor mit einer Dicke im Bereich von 2 bis 50 nm insbesondere 3 bis 8 nm und einer Länge im Bereich von 5 bis 150 nm, insbesondere 10 bis 40 nm. Unter Dicke ist hier der kleinste Durchmesser der Stäbchen zu verstehen, unter Länge ihr größter Durchmesser.

Gemäß einer bevorzugten Ausführungsform der Erfindung weisen die nanopartikulären Primärteilchen stäbchenförmige Kristalle mit einem mittleren Länge-zu-Breite-Verhältnis des Kristlls von 3 bis < 5, insbesondere von ca. 4, auf. Unter mittleren Länge-zu-Breite-Verhältnis ist erfindungsgemäß zu verstehen, dass ein Großteil der Kristalle ein Länge-zu-Breite-Verhältnis in dem angegebenen Bereich aufweist.

Die Bestimmung des Länge-zu-Breite Verhältnisses erfolgt ebenfalls über das Röntgenbeugungsverfahren.

Die räumliche Struktur der erfindungsgemäßen Kompositmaterialien aus einer Proteinkomponente sowie den schwerlöslichen nanopartikulären Calciumsalzen wird am Beispiel der in Abbildung 1 dargestellten TEM-Aufnahme eines Kompositmaterials aus Hydroxylapatit und Gelatine Typ A deutlich (200.000-fache Vergrößerung; 1 cm in der Abbildung entspricht 40 nm). Der hochmolekularen Proteinkomponente, die eine im wesentlichen durch ihre Aminosäuresequenz bestimmte dreidimensionale Struktur einnimmt, sind die stäbchenförmigen Nanopartikel aus Hydroxylapatit aufgelagert, die Nanopartikel bilden also gewissermaßen die räumliche Struktur der Proteinkomponente ab. Dies wird deutlich anhand von Abbildung 2, welche eine TEM-Aufnahme des Gelatine Typ A - Gerüsts des gleichen Kompositmaterials nach Herauslösen des Hydroxylapatits mittels einer Lösung von Ethylendiamintetraacetat zeigt (56.000-fache Vergrößerung; 1,1 cm in der Abbildung entspricht 200 nm). Die Art und Weise der Anlagerung der anorganischen Partikel an das Grundgerüst der Proteinkomponente wird durch die Primärstruktur (Aminosäuresequenz) sowie je nach der Natur der Proteinkomponente ihrer Sekundär-, Tertiär- und Quartärstruktur bestimmt. Überraschenderweise wurde gefunden, daß die räumliche Verteilung und der quantitative Umfang der Anlagerung der anorganischen Nanopartikel an die Proteinkomponente durch die Art und Menge der in der Proteinkomponente vorliegenden Aminosäuren und damit durch die Auswahl der Proteinkomponenten beeinflußt werden kann. So kann beispielsweise durch die Auswahl von Proteinkomponenten, die reich an den Aminosäuren, Asparaginsäure, Glutaminsäure oder Cystein sind, eine besonders hohe Beladung mit dem schwerlöslichen Calciumsalz erreicht werden. Je nach der räumlichen Verteilung dieser Aminosäuren im Proteingerüst kann darüber hinaus eine räumlich in bestimmter Weise strukturierte Beladung der Proteinkomponente mit dem schwerlöslichen Calciumsalz erreicht werden.

Die erfindungsgemäßen Kompositmaterialien sind also strukturierte Kompositmaterialien im Gegensatz zu dem bei R. Z. Wang et al. beschriebenen Komposit aus Hydroxylapatit und Kollagen, in welchem gleichmäßig verteilte Hydroxylapatit-Nanopartikel vorliegen. Ein weiterer wesentlicher Unterschied zwischen dem Gegenstand der vorliegenden Erfindung und dem Stand der Technik besteht in der Größe und Morphologie der anorganischen Komponente. Die in dem von R. Z.

Wang et al. beschriebenen Hydroxylapatit-Kollagen-Komposit vorliegenden Hydroxylapatit-Teilchen haben eine Größe von 2-10 nm. Hydroxylapatit-Partikel in diesem Größenbereich sind dem Bereich der amorphen oder teilweise röntgenamorphen Stoffe zuzurechnen.

Überraschenderweise gelang es mit der vorliegenden Erfindung, Kompositmaterialien mit kristallinen anorganischen Nanopartikeln zu erzeugen, in welchen die Nanopartikel eine mikroskopisch deutlich erkennbare kristalline Morphologie aufweisen. Abbildung 1 zeigt die stäbchenförmige Struktur der anorganischen Nanopartikel. Es wurde weiterhin gefunden, daß die erfindungsgemäßen strukturierten Kompositmaterialien im Gegensatz zum Stand der Technik zu einen besonders effektiven Biomineralisationsprozeß führen. Es wird angenommen, daß dies mit der Mikrostruktur des Kompositmaterials und insbesondere der Größe und Morphologie der Calciumsalz-Kristalle zusammenhängt. So wird angenommen, daß die Längsachse der Calciumsalz-Nanopartikel eine Vorzugsrichtung für das weitere Kristallwachstum während der Biomineralisation darstellt.
Als in Wasser schwerlöslich sollen solche Salze verstanden werden, die bei 20 °C zu weniger als 1 g/l löslich sind. Bevorzugt geeignete Calciumsalze sind Calciumhydroxyphosphat (Ca₅[OH(PO₄)₃]) bzw. Hydroxylapatit, Calciumfluor-phosphat (Ca₅[F(PO₄)₃]) bzw. Fluorapatit, Fluor-dotierter Hydroxylapatit der allgemeinen Zusammensetzung Ca₅(PO₄)₃(OH,F) und Calciumfluorid (Ca F₂) bzw. Fluorit (Flußspat); insbesondere bevorzugt sind Hydroxylapatit und/oder Fluorapatit.

Als Calciumsalz kann in den erfindungsgemäßen Kompositmaterialien eines oder auch mehrere Salze im Gemisch, ausgewählt aus der Gruppe von Phosphaten, Fluoriden und Fluorophosphaten, die wahlweise zusätzlich Hydroxyl- und/oder Carbonat-Gruppen enthalten können, im Gemisch enthalten sein.

Als Proteine kommen im Rahmen der vorliegenden Erfindung grundsätzlich alle Proteine unabhängig von ihrem Ursprung oder ihrer Herstellung in Betracht. Beispiele für Proteine tierischen Ursprungs sind Keratin, Elastin, Kollagen, Fibroin, Albumin, Casein, Molkeprotein, Plazentaprotein. Erfindungsgemäß bevorzugt aus diesen sind Kollagen, Keratin, Casein, Molkeprotein, Proteine pflanzlichen Ursprungs wie beispielsweise Weizen- und Weizenkeimprotein, Reisprotein, Sojaprotein, Haferprotein, Erbsenprotein, Kartoffelprotein, Mandelprotein und Hefeprotein können erfindungsgemäß ebenfalls bevorzugt sein.

Unter Proteinhydrolysaten sind im Rahmen der vorliegenden Erfindung Abbauprodukte von Proteinen wie beispielsweise Kollagen, Elastin, Casein, Keratin, Mandel-, Kartoffel-, Weizen-, Reis- und Sojaprotein zu verstehen, die durch saure, alkalische und / oder enzymatische Hydrolyse der Proteine selbst oder ihrer Abbauprodukte wie beispielsweise Gelatine erhalten werden. Für den enzymatischen Abbau sind alle hydrolytisch wirkenden Enzyme geeignet, wie z. B. alkalische Proteasen. Weitere geeignete Enzyme sowie enzymatische Hydrolyseverfahren sind beispielsweise beschrieben in K. Drauz und H. Waldmann, Enzyme Catalysis in Organic Synthesis, VCH-Verlag, Weinheim 1975. Bei dem Abbau werden die Proteine in kleinere Untereinheiten gespalten, wobei der Abbau über die Stufen der Polypeptide über die Oligopeptide bis hin zu den einzelnen Aminosäuren gehen kann. Zu den wenig abgebauten Proteinhydrolysaten zählt beispielsweise die im Rahmen der vorliegenden Erfindung bevorzugte Gelatine, welche Molmassen im Bereich von 15000 bis 250000 D aufweisen kann. Gelatine ist ein Polypeptid, das vornehmlich durch Hydrolyse von Kollagen unter sauren (Gelatine Typ A) oder alkalischen (Gelatine Typ B) Bedingungen gewonnen wird. Die Gelstärke der Gelatine ist proportional zu ihrem Molekulargewicht, d. h., eine stärker hydrolysierte Gelatine ergibt eine niedriger viskose Lösung. Die Gelstärke der Gelatine wird in Bloom-Zahlen angegeben. Bei der enzymatischen Spaltung der Gelatine wird die Polymergröße stark erniedrigt, was zu sehr niedrigen Bloom-Zahlen führt.

Weiterhin sind im Rahmen der vorliegenden Erfindung als Proteinhydrolysate bevorzugt die in der Kosmetik gebräuchlichen Proteinhydrolysate mit einem durchschnittlichen Molekulargewicht im Bereich von 600 bis 4000, besonders bevorzugt von 2000 bis 3500. Übersichten zu Herstellung und Verwendung von Proteinhydrolysaten sind beispielsweise von G. Schuster und A. Domsch in Seifen Öle Fette Wachse **108,** (1982) 177 bzw. Cosm.Toil. **99,** (1984) 63, von H. W. Steisslinger in Parf.Kosm. **72,** (1991) 556 und F. Aurich et al. in Tens.Surf.Det. **29,** (1992) 389 erschienen. Vorzugsweise werden erfindungsgemäß Proteinhydrolysate aus Kollagen, Keratin, Casein sowie pflanzlichen Proteinen eingesetzt, beispielsweise solche auf Basis von Weizengluten oder Reisprotein, deren Herstellung in den beiden Deutschen Patentschriften DE 19502167 C1 und DE 19502168 C1 (Henkel) beschrieben wird.

Unter Proteinhydrolysat-Derivaten sind im Rahmen der vorliegenden Erfindung chemisch und / oder chemoenzymatisch modifizierte Proteinhydrolysate zu verstehen wie beispielsweise die unter den INCl-Bezeichnungen Sodium Cocoyl Hydrolyzed Wheat Protein, Laurdimonium Hydroxypropyl Hydrolyzed Wheat Protein, Potassium Cocoyl Hydrolyzed Collagen, Potassium Undecylenoyl Hydrolyzed Collagen und Laurdimonium Hydroxypropyl Hydrolyzed Collagen bekannten Verbindungen. Vorzugsweise werden erfindungsgemäß Derivate aus Proteinhydrolysaten des Kollagens, Keratins und Caseins sowie pflanzlichen Proteinhydrolysaten eingesetzt wie z. B. Sodium Cocoyl Hydrolyzed Wheat Protein oder Laurdimonium Hydroxypropyl Hydrolyzed Wheat Protein.

Weitere Beispiele für Proteinhydrolysate und Proteinhydrolysat-Derivate, die unter den Rahmen der vorliegenden Erfindung fallen, sind beschrieben in CTFA 1997 International Buyers' Guide, John A. Wenninger et al. (Ed.), The Cosmetic, Toiletry, and Fragrance Association, Washington DC 1997, 686-688.

Die Proteinkomponente kann in jedem der erfindungsgemäßen Kompositmaterialien durch einen oder mehrere Stoffe ausgewählt aus der Gruppe von Proteinen, Proteinhydrolysaten und Proteinhydrolysat-Derivaten gebildet werden.

Als Proteinkomponenten bevorzugt sind alle strukturbildenden Proteine, Proteinhydrolysate und Proteinhydrolysat-Derivate, worunter solche Proteinkomponenten zu verstehen sind, die aufgrund ihrer chemischen Konstitution bestimmte dreidimensionale räumliche Strukturen ausbilden, die dem Fachmann aus der Proteinchemie unter den Begriffen Sekundär-, Tertiär- oder auch Quartärstruktur geläufig sind.

Zur Herstellung des Kompositmaterials wird ausdrücklich auf die Offenbarung der WO 01/01930 A1 verwiesen.

Der Gehalt des Kompositmaterials in den erfindungsgemäßen Mund- und Zahnpflegemitteln beträgt 0,01 bis 10 Gew.-%, bevorzugt 0,1 bis 2 Gew.-%, bezogen auf das Gesamtgewicht des Mittels.

Die erfindungsgemäßen Mund- und Zahnpflegemittel enthalten weiterhin 10 bis 35 Gew.-%, insbesondere 10 bis 25 Gew.-% eines Putzkörpergemisches, wovon 0,01 bis 5 Gew.-% des Putzkörpergemisches aus dem Poliermittel Aluminiumoxid bestehen.

Putzmittel gehören zu den essentiellen Bestandteilen einer Zahncreme und liegen je nach ihrer beabsichtigten Funktion allein oder in Kombination mit anderen Putzkörpern vor. Sie dienen zur mechanischen Entfernung des unverkalkten Zahnbelags und sollen idealerweise zur Glanzgebung der Zahnoberfläche (Poliereffekt) bei gleichzeitiger minimaler Scheuerwirkung (Abrasionseffekt) und Schädigung des Zahnschmelzes und des Dentins führen.

Das Abrasionsverhalten der Putzkörper wird im Wesentlichen durch deren Härte, Korngrößenverteilung und Oberflächenstruktur bestimmt. Bei der Auswahl geeigneter Putzkörper werden folglich insbesondere solche bevorzugt ausgewählt, die bei hoher Reinigungsleistung über eine möglichst geringe Abrasionswirkung verfügen.
Heutzutage werden als Putzkörper vorwiegend Stoffe verwendet, die kleine Korngrößen aufweisen, weitgehend frei von scharfen Ecken und Kanten sind und über eine geeignete Härte verfügen.

Da die Deponierung des Kompositmaterials gewährleistet sein und auch nach dem Putzvorgang erhalten bleiben soll, werden als weitere Putzmittel gezielt solche mit besonders niedriger Abrasivität und guter Putzwirkung ausgewählt. Geeignete weitere Putzmittel im Sinne der Erfindung weisen daher eine mittlere Korngröße von 1 - 200 µm, vorzugsweise 1 - 50 µm und insbesondere 1 - 10 µm auf.

Prinzipiell können die erfindungsgemäßen Putzmittel ausgewählt sein aus Kieselsäuren, Aluminiumhydroxid, Aluminiumoxid, Silikaten, organischen Polymeren oder Gemischen davon. Weiterhin können aber auch sogenannte Metaphosphate, Erdalkalimetallcarbonate oder -hydrogencarbonate sowie calciumhaltige Polierkomponenten in den erfindungsgemäßen Mitteln enthalten sein.

Es kann erfindungsgemäß bevorzugt sein, Kieselsäuren als Putzmittel in Zahnpasten oder flüssigen Zahnreinigungsmitteln einzusetzen. Man unterscheidet unter den Kieselsäure-Putzmitteln grundsätzlich zwischen Gelkieselsäuren, Hydrogelkieselsäuren und Fällungskieselsäuren. Fälluns- und Gelkieselsäuren sind erfindungsgemäß besonders bevorzugt, da sie bei ihrer Herstellung breit variiert werden können und besonders gut mit Fluorid-Wirkstoffen verträglich sind. Sie eigenen sich weiterhin auch besonders gut für die Herstellung von Gel- oder Liquid-Zahncremes.

Gelkieselsäuren werden durch die Umsetzung von Natriumsilikatlösungen mit starken, wäßrigen Mineralsäuren unter Ausbildung eines Hydrosols, Alterung zum Hydrogel, Waschen und anschließendem Trocknen erzeugt. Erfolgt die Trocknung unter schonenden Bedingungen auf Wassergehalte von 15 bis 35 Gew.-%, so werden sogenannte Hydrogelkieselsäuren erhalten, wie sie beispielsweise auch in der US 4,153,680 beschrieben sind. Durch Trocknung dieser Hydrogelkieselsäuren auf Wassergehalte unterhalb von 15 Gew.-% erfolgt eine irreversible Schrumpfung der vorher lockeren Struktur zur dichten Struktur des sogenannten Xerogels. Solche Xerogelkieselsäuren sind beispielsweise aus der US 3,538,230 bekannt.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei denen die Aggregation zum Sol und Gel nicht eintreten kann. Geeignete Verfahren zur Herstellung von Fällungskieselsäuren sind beispielsweise in der DE-OS 25 22 586 und in der DE-OS 31 14 493 beschrieben. Erfindungsgemäß besonders geeignet ist eine gemäß der DE-OS 31 14 493 hergestellte Fällungskieselsäure mit einer BET-Oberfläche von 15 - 110 m²/g, einer Partikelgröße von 0,5 bis 20 µm, wobei wenigstens 80 Gew.-% der Primärpartikel unter 5 µm liegen sollen, und einer Viskosität in 30%iger Glycerin-Wasser-(1:1)-Dispersion von 30 - 60 Pa s (20°C) in einer Menge von 10 - 20 Gew.-% der Zahnpaste. Bevorzugt geeignete Fällungskieselsäuren dieser Art weisen außerdem gerundete Ecken und Kanten auf und sind beispielsweise unter der Handelsbezeichnung Sident^{®}12 DS der Firma Degussa erhältlich.

Weitere Fällungskieselsäuren dieser Art sind Sident^{®}8 der Firma Degussa und Sorbosil^{®}AC 39 der Firma Crosfield Chemicals. Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa s aufweisen.

Des weiteren können die Kieselsäuren vom Typ Zeodent^{®} der Firma Huber-Corp., Tixosil® der Firma Rhodia sowie weitere Sorbosil-Typen in den erfindungsgemäßen Mitteln eingesetzt werden. Besonders bevorzugt sind Zeodent^{®}113, Tixosil^{®} 123 und 73 sowie Sorbosil^{®}AC39.

Zahnpasten, die eine deutlich höhere Viskosität von mehr als 100 Pa s (25°C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 bis 3 µm. Solchen Zahnpasten setzt man daher bevorzugt neben den genannten Fällungskieselsäuren noch feinteiligere, sogenannte Verdickungskieselsäuren mit einer BET-Oberfläche von 150 - 250 m²/g zu. Als Beispiele für Handelsprodukte, die die genannten Bedingungen erfüllen, sind insbesondere Sipernat^{®}22 LS oder Sipernat^{®}320 DS der Firma Degussa zu nennen.

Als Aluminiumoxid-Poliermittel eignet sich bevorzugt eine schwach calcinierte Tonerde mit einem Gehalt an α- und γ-Aluminiumoxid in einer Menge von etwa 0,01 bis 5 Gew.-%, bevorzugt 0,1 bis 2 Gew.-%, bezogen auf das Gesamtgewicht des Mittels.

Geeignete schwach calcinierte Tonerden werden durch Calcination aus Aluminiumhydroxid hergestellt. Aluminiumhydroxid geht durch Calcination in das bei Temperaturen oberhalb 1200°C thermodynamisch stabile α-Al₂O₃ über. Die bei Temperaturen zwischen 400 und 1000°C auftretenden, thermodynamisch instabilen Al₂O₃-Modifikationen bezeichnet man als Gamma-Formen (vgl. Ullmann, Enzyclopädie der technischen Chemie, 4. Auflage (1974), Band 7, Seite 298). Durch Wahl der Temperatur und der Zeitdauer bei der Calcination kann man den Calcinationsgrad, d.h. die Umwandlung in das thermodynamisch stabile α-Al₂O₃ auf beliebige Höhe einstellen. Man erhält durch schwache Calcination eine Tonerde mit einem Gehalt an γ-Al₂O₃, der um so niedriger ist, je höher die Calcinationstemperatur und je länger die Calcinationsdauer gewählt wird. Schwach calcinierte Tonerden unterscheiden sich von reinem α-Al₂O₃ durch eine geringere Härte der Agglomerate, eine größere spezifische Oberfläche und größere Porenvolumina.

Der Dentinabrieb (RDA) der erfindungsgemäß zu verwendenden schwächer calcinierten Tonerden mit einem Anteil von 10 - 50 Gew.-% γ-Al₂O₃ beträgt nur 30 - 60 % des Dentinabriebs eines stark calcinierten, reinen α-Al₂O₃ (gemessen in einer Standard-Zahnpaste mit 20 Gew.-% Tonerde als einzigem Poliermittel).

Aluminiumoxid-Poliermittel verschiedener Calcinationsgrade, Mahlfeinheit und Schüttgewichte sind im Handel erhältlich, z.B. die "Poliertonerden" der Firma Giulini-Chemie beziehungsweise ALCOA. Eine bevorzugt geeignete Qualität "Poliertonerde P10 feinst" weist eine Agglomeratgröße unter 20 µm, eine mittlere Primärkristallgröße von 0,5 - 1,5 µm und ein Schüttgewicht von 500 - 600 g/l auf.

Die Verwendung von Silikaten als Poliermittelkomponenten kann ebenfalls erfindungsgemäß bevorzugt sein. Sie werden insbesondere in der modernen Praxis als Putzkörper eingesetzt. Beispiele für erfindungsgemäß einsetzbare Silikate sind Aluminiumsilikate und Zirkoniumsilikate. Insbesondere das Natrium-Aluminiumsilikat der empirischen Formel Na₁₂(AlO₂)₁₂(SiO₂)₁₂ x 7H₂O kann als Poliermittel geeignet sein, wie beispielsweise das synthetische Zeolith A.

Beispiele für erfindungsgemäße wasserunlösliche Metaphosphate sind vor allem Natriummetaphosphat, Calciumphosphat wie beispielsweise Tricalciumphosphat, Calciumhydrogenphosphat, Calciumhydrogenphosphat-Dihydrat und Calciumpyrophosphat.

Des weiteren können erfindungsgemäß Magnesiumcarbonat, Magnesiumhydrogenphosphat, Trimagnesiumphosphat oder Natriumhydrogencarbonat als Poliermittel, insbesondere als Mischung mit anderen Poliermitteln, eingesetzt werden.

Ein weiteres Poliermittel, das sich für den Einsatz in den erfindungsgemäßen Mund- und Zahnpflegemitteln eignet, ist Calciumphosphat-dihydrat (CaHPO₄ x 2H₂O). Calciumphosphat-dihydrat kommt in der Natur als Brushit vor und ist als Poliermittel im Handel in geeigneten Korngrößen von 1 bis 50 µm erhältlich.

Der Gesamt-RDA-Wert des Mund- und Zahnpflegemittels beträgt erfindungsgemäß 50 bis 170, insbesondere 60 bis 120.

Die gezielte Einstellung des RDA-Wertes bei den erfindungsgemäßen Mund- und Zahnpflegemitteln gewährleistet eine schonende aber effektive Reinigung des Mundraumes, wodurch auch eine geringere Kratzwirkung auf der Zahnhartsubstanz (Zahnschmelz) erzielt werden kann. Des weiteren ist die Beachtung der Abrasivität eines Zahnreinigungsmittels auf dem Zahnbein (Dentin) zu beachten, denn dies ist deutlich weicher als der Zahnschmelz und kann insbesondere bei freiliegenden Zahnhälsen zu Schmerzempfindlichkeit führen.
Der RDA-Wert kann daher als Kennung für die Schonung eines Zahnreinigungsmittels herangezogen werden. Erfindungsgemäß wurde durch geeignete Wahl des Putzkörpergemisches ein RDA-Wet eingestellt, mit dem eine schonende aber gründliche Reinigung der Zähne möglich ist, der aber dennoch nicht der gleichzeitigen Deponierung der remineralisierenden Komponente entgegensteht.

Der RDA-Wert wird nach der Methode von Hefferren, Journal of Dental Research, July-August (1976), Seite 563-573; US-A-4,340,583; US-A-4,420,312 und US-A-4,421,527, bestimmt. Dabei wird mittels Elektronenbestrahlung der Versuchszahn radioaktiv markiert, anschließend mit einer definierten Zahnpastenaufschlämmung gebürstet, die Radioaktivität des abgeriebenen Zahnmaterials gemessen und mit dem RDA-Wert einer Standardzahnpaste verglichen.

Es kann erfindungsgemäß bevorzugt sein, den durch das Kompositmaterial bedingten Remineralisationsprozess durch die Zugabe eines Remineralisationsförderungsmittels noch weiter zu unterstützen. Solche Remineralisationsförderungsmittel werden den Mund- und Zahnpflegemitteln üblicherweise in Mengen von 0,1 bis 10 Gew.-%, vorzugsweise 0,1 bis 5 Gew.-% und insbesondere 0,1 bis 3 Gew.-%, jeweils bezogen auf das Gesamtgewicht des Mittels, beigemischt.

Die Remineralisationsförderungs-Komponente fördert in den erfindungsgemäßen Mitteln die Remineralisierung des Zahnschmelzes und die Verschließung von Dentalläsionen und ist ausgewählt aus Fluoriden, mikropartikulären Phosphatsalzen des Calciums wie z. B. Calciumglycerinphosphat, Calciumhydrogenphopsphat, Hydroxylapatit, Fluorapatit, F-dotierter Hydroxylapatit, Dicalciumphosphat-Dihydrat sowie Calciumfluorid. Aber auch Magnesiumsalze wie z. B. Magnesiumsulfat, Magnesiumfluorid oder Magnesiummonofluorophosphat wirken remineralisierend.

In einer bevorzugten Ausführungsform der Erfindung wird als Remineralisationsförderungsmittel ein Magnesiumsalz eingesetzt.

Geeignete Ausführungsformen des erfindungsgemäßen Mund- und Zahnpflegemittels sind feste, flüssige oder halbflüssige Zahnpasten und Zahngele.

Die erfindungsgemäßen Mund- und Zahnpflegemittel enthalten gemäß einer weiteren bevorzugten Ausführungsform zusätzliche Zahnpasteninhaltsstoffe wie Tenside, Feuchthaltemittel, Bindemittel, Geschmacksstoffe und Wirkstoffe gegen Zahn- und Zahnfleischerkrankungen.

Für eine Verbesserung der Reinigungswirkung und der Schaumbildung der erfindungsgemäßen Mund- und Zahnpflegemittel werden üblicherweise oberflächenaktive Tenside oder Tensidgemische eingesetzt. Sie fördern die schnelle und vollständige Auflösung und Verteilung von Zahncremes in der Mundhöhle und unterstützen gleichzeitig die mechanische Zahnbelagsentfernung, insbesondere an den Stellen, die mit einer Zahnbürste nur schwer zugänglich sind. Darüber hinaus begünstigen sie die Einarbeitung wasserunlöslicher Stoffe, beispielsweise von Aromaölen, stabilisieren die Poliermitteldispersion und unterstützen die Antikarieswirkung von Fluoriden.

Prinzipiell können anionische Tenside, zwitterionische- und ampholytische Tenside, nichtionogene Tenside, kationische Tenside oder Gemische dieser Verbindungen als Tenside in Zahncremeformulierungen verwendet werden. Erfindungsgemäß enthalten Zahncremes vorzugsweise mindestens ein Tensid aus der Gruppe der anionischen Tenside.

Das Tensid oder das Tensidgemisch wird in den erfindungsgemäßen Zusammensetzungen üblicherweise in einer Menge von 0,1 - 10 Gew.-%, vorzugsweise 0,3 - 7 Gew.-% und insbesondere 1 - 5 Gew.-%, bezogen auf das Gesamtgewicht der Zusammensetzung, eingesetzt.

### Anionische Tenside

Geeignete Tenside mit guter Schaumwirkung sind anionische Tenside, die auch eine gewisse enzymhemmende Wirkung auf den bakteriellen Stoffwechsel des Zahnbelags aufweisen.

Hierzu gehören beispielsweise Alkali- oder Ammoniumsalze, insbesondere Natriumsalze, von C₈-C₁₈-Alkancarbonsäuren, von Alkylpolyglycolethersulfaten mit 12 - 16 C-Atomen in der linearen Alkylgruppe und 2 - 6 Glycolethergruppen im Molekül, von linearen Alkan-(C₁₂-C₁₈)-sulfonaten, Sulfobernsteinsäuremonoalkyl-(C₁₂-C₁₈)-estern, sulfatierten Fettsäuremonoglyceriden, sulfatierten Fettsäurealkanolamiden, Sulfoessigsäurealkyl-(C₁₂-C₁₆)-estern, Acylsarcosinen, Acyltauriden und Acylisethionaten mit jeweils 8 - 18 C-Atomen in der Acylgruppe.

Bevorzugt ist die Verwendung mindestens eines anionischen Tensids, insbesondere eines Natriumlaurylalkylsulfats mit 12 - 18 C-Atomen in der Alkylgruppe. Ein derartiges Tensid ist Natriumlaurylsulfat, das beispielsweise unter der Bezeichnung Texapon^{®}K12 G im Handel erhältlich ist.

### Zwitterionische und ampholytische Tenside

Es kann erfindungsgemäß bevorzugt sein, zwitterionische und/oder ampholytische Tenside, bevoruzugt in Kombination mit anionischen Tensiden, einzusetzen. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Trimethylammoniumglycinat, Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA Bezeichnung Cocamidopropyl Betaine bekannte Fettsäureamid-Derivat. Solche Produkte sind beispielsweise unter der Bezeichnung Tego-Betain^{®}BL 215 und ZF 50 sowie Genagen^{®}CAB im Handel erhältlich.

Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N- Alkyliminodi propionsäuren, N-Hydroxyethyl-N-alkylamidopropylglycine, N-Alkyltaurine, N- Alkyisarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin. Neben den ampholytischen kommen auch quartäre Emulgatoren in Betracht, wobei solche vom Typ der Esterquats, vorzugsweise methyl-quaternierte Difettsäu- retriethanolaminester-Salze, besonders bevorzugt sind.

### Nichtionogene Tenside

Erfindungsgemäß besonders geeignet zur Unterstützung der Reinigungswirkung sind nichtionogene Tenside. Insbesondere bevorzugt sind diejenigen nichtionogenen Tenside, die aus mindestens einer der folgenden Gruppen ausgewählt sind:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C- Atomen in der Alkylgruppe;
- C₁₂-C₁₈- Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analogas;
- Anlagerungsprodukte von 15 bis 60 Mol Ethylenoxid an Ricinusöl und/oder gehärtetes Ricinusöl;
- Polyol- und insbesondere Polyglycerinester, wie z.B. Polyglycerinpolyricinoleat, Polyglycerinpoly-12-hydroxystearat oder Polyglycerindimerat.

Ebenfalls geeignet sind Gemische von Verbindungen aus mehreren dieser Substanzklassen;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Sucrose, Alkylglucoside (z.B. Methylglucosid, Butylglucosid, Laurylglucosid) sowie Polyglucoside (z.B. Cellulose);
- Mono-, Di- und Trialkylphosphate sowie Mono-, Di- und/oder Tri- PEGalkylphosphate und deren Salze;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
- Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar und sind erfindungsgemäß bevorzugt. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C₁₂-C₁₈-Fettsäuremono- und - diester von Anlagerungsprodukten von Ethylenoxid an Glycerin sind aus DE-PS 2024051 als Rückfettungsmittel für kosmetische Zubereitungen bekannt.
C₈-C₁₈-Alkylmono- und -oligoglycoside, ihre Herstellung und ihre Verwendung sind aus dem Stand der Technik, beispielsweise aus US-A-3,839,318, DE-A-20 36 472, EP-A-77 167 oder WO-A-93/10132 bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Bevorzugt eignet sich als Alkyl-(oligo)-glycosid ein Alkyl-(oligo)-glycosid der Formel RO(C₆H₁₀O)ₓ-H, in der R für eine Alkylgruppe mit 12 bis 14 C-Atomen steht und x einen Mittelwert von 1 bis 4 aufweist.

Als besonders bevorzugtes Beispiel eines erfindungsgemäß einsetzbaren, nichtionogenen Tensids ist beispielsweise das PEG-Glycerylstearat zu nennen, das unter der Bezeichnung Tagat^{®}S im Handel erhältlich ist.

Feuchthaltemittel werden in der Zahnkosmetik üblicherweise zum Schutz vor Austrocknung sowie zur Konsistenzregelung und Kältestabilität der Produkte eingesetzt. Sie können aber ferner auch zur Suspensionsvermittlung und zur Geschmacks- oder Glanzbeeinflussung dienen.

Gewöhnlich werden als Feuchthaltemittel toxikologisch unbedenkliche Polyole, wie beispielsweise Sorbitol, Xylitol, Glycerin, Mannitol, 1,2-Propylenglycol oder Gemische davon verwendet, aber auch Polyethylenglycole mit Molekulargewichten von 400 - 2000 können als Feuchthaltemittelkomponenten in Zahncremes dienen.

Bevorzugt ist die Kombination mehrerer Feuchthaltemittelkomponenten, wobei die Kombination von Glycerin und Sorbitol mit einem Gehalt an 1,2-Propylenglycol oder Polyethylenglycol als besonders bevorzugt anzusehen ist.

Je nach Produkttyp ist das Feuchthaltemittel oder das Gemisch aus Feuchthaltemitteln in der Gesamtzusammensetzung in einer Menge von 10 - 85 Gew.-%, vorzugsweise 15 - 70 Gew.-% und insbesondere 25 - 50 Gew.-% enthalten.

Die erfindungsgemäßen Mittel enthalten in einer bevorzugten Ausführungsform zusätzlich mindestens ein Binde- oder Verdickungsmittel. Diese wirken konsistenzregulierend und verhindern weiterhin die Separation der flüssigen und festen Bestandteile.

Ihre Einsatzmengen in den erfindungsgemäßen Zusammensetzungen betragen 0,1 - 5 Gew.-%, vorzugsweise 0,1 - 3 Gew.-% und insbesondere 0,5 - 2 Gew.-%.

Verwendet werden erfindungsgemäß beispielsweise natürliche und/oder synthetische wasserlösliche Polymere wie Alginate, Carrageenane, Agar-Agar, Guar-Gum, Gummi arabicum, Succinoglycan-Gum, Guarmehl, Johannisbrotkernmehl, Tragant, Karaya-Gummi, Xanthan, Pektine, Cellulose und deren ionogene und nicht-ionogene Derivate wie beispielsweise Carboxymethylcellulose, Hydroxyethylcellulose oder Methylhydroxypropyl-cellulose, hydrophob modifizierte Cellulosen, Stärke- und Stärkeether.

Auch wasserlösliche Carboxyvinylpolymere (z.B. Carbopol^{®}-Typen), Polyvinylalkohol, Polyvinylpyrrolidon und höhermolekulare Polyethylenglycole (insbesondere solche mit Molekulargewichten von 10² - 10⁶ D) eignen sich als Binde- oder Verdickungsmittel. Ebenso können Schichtsilikate und feinteilige Kieselsäuren (Aerogelkieselsäuren und pyrogene Kieselsäuren) diese Funktion erfüllen.

In einer weiteren bevorzugten Ausführungsform enthält das erfindungsgemäße Mund- und Zahnreinigungsmittel zusätzliche Wirkstoffe gegen Zahn- und Zahnfleischerkrankungen. Unter solchen Wirkstoffen sind erfindungsgemäß Antikaries-wirkstoffe, antimikrobielle Wirkstoffe, Zahnstein-Inhibitoren, Geschmacksstoffe oder eine beliebige Kombination dieser Stoffe zu verstehen.

### Antikaries-Wirkstoffe

Zur Bekämpfung von und Vorbeugung gegen Karies eignen sich vor allem Fluorverbindungen, bevorzugt aus der Gruppe der Fluoride oder Monofluorophosphate in einer Menge von 0,1 - 0,5 Gew.-% Fluor. Geeignete Fluorverbindungen sind z. B. Natriumfluorid, Kaliumfluorid, Zinnfluorid, Dinatriummonofluorophosphat (Na₂PO₃F), Dikaliummonofluorophosphat oder das Fluorid einer organischen Aminoverbindung.

### Antimikrobielle Wirkstoffe

Als antimikrobielle Komponente eignen sich z. B. Phenole, Resorcine, Bisphenole, Salicylanilide und -amide sowie deren halogenierte Derivate, halogenierte Carbanilide und p-Hydroxybenzoesäureester.

Unter den antimikrobiellen Komponenten sind diejenigen besonders geeignet, die das Wachstum von Plaque-Bakterien hemmen. Beispielsweise sind halogenierte Diphenylether, wie 2,4-Dichlor-2'-hydroxydiphenylether, 4,4'-Dichlor-2'-hydroxydiphenylether, 2,4,4'-Tribrom-2'-hydroxydiphenylether, 2,4,4'-Trichlor-2'-hydroxydiphenylether (Triclosan) als antimikrobielle Wirkstoffe geeignet. Neben Bromchlorophen, Bisbiguaniden wie Chlorhexidin und Alexidin, Phenylsalicylsäureestern und 5-Amino-1,3-bis(2-ethylhexyl)-hexahydro-5-methylpyrimidin (Hexetidin) wirken auch Zink- und Kupferionen antimikrobiell, wobei synergistische Effekte insbesondere in Kombination mit Hexetidin und Triclosan auftreten. Auch quartäre Ammoniumverbindungen, wie z. B. Cetylpyridiniumchlorid, Benzalkoniumchlorid, Domiphenbromid und Dequaliniumchlorid sind einsetzbar. Als antimikrobiell wirksam haben sich auch Octapinol, Octenidine und Sanguinarin erwiesen.

Die antimikrobiellen Wirkstoffe werden bevorzugt in Mengen von 0,01 - 1 Gew.-% in den erfindungsgemäßen Mitteln eingesetzt. Besonders bevorzugt wird Irgacare^{®} MP in einer Menge von 0,01 - 0,3 Gew.-% verwendet.

### Zahnsteininhibitoren

Bei Zahnstein handelt es sich um Mineralablagerungen, die dem natürlichen Zahnschmelz sehr ähnlich sind. Um eine Zahnsteinbildung zu inhibieren, werden den erfindungsgemäßen Zahnreinigungsmitteln Stoffe zugesetzt, die gezielt in die Kristallkeimbildung eingreifen und bereits vorhandene Keime am Weiterwachsen hindern. Hierbei handelt es sich beispielsweise um kondensierte Phosphate, die bevorzugt gewählt werden aus der Gruppe der Tripolyphosphate, der Pyrophophate, der Trimetaphosphate oder deren Gemischen. Sie werden in Form ihrer Alkali- oder Ammoniumsalze, bevorzugt in Form ihrer Natrium- oder Kaliumsalze eingesetzt. Wäßrige Lösungen dieser Phosphate reagieren typischerweise alkalisch, so daß der pH-Wert der erfindungsgemäßen Zahnpflegemittel ggf. durch Zusatz von Säure auf Werte von 7,5 - 9 eingestellt wird. Als Säuren können dabei z. B. Zitronensäure, Phosphorsäure oder saure Salze, z. B. NaH₂PO₄ verwendet werden. Der gewünschte pH-Wert des Zahnpflegemittels kann aber auch durch Zusatz saurer Salze der kondensierten Phosphate, also z. B. K₂H₂P₂O₇, eingestellt werden.

Auch Gemische verschiedener kondensierter Phosphate und/oder hydratisierte Salze der kondensierten Phosphate sind erfindungsgemäß einsetzbar. Zahnsteininhibitoren werden üblicherweise in Mengen von 0,1 - 5 Gew.-%, bevorzugt 0,1 - 3 Gew.-% und insbesondere 0,1 - 2 Gew.-% in den erfindungsgemäßen Mitteln eingesetzt.

Weitere geeignete Zahnsteininhibitoren sind Organophosphonate wie 1-Azacycloheptan-2,2-diphosphonat (Na-Salz), 1-Hydroxyethan-1,1-diphosphonat (Na-Salz) und Zinkcitrat.

### Wirkstoffe gegen hypersensible Zähne

Vorzugsweise enthalten die erfindungsgemäßen Mittel weiterhin Wirkstoffe gegen hypersensible Zähne, sie ausgewählt sind aus Kalium- und Strontiumsalzen wie Kaliumchlorid, Kaliumsulfat, Kaliumbicarbonat, Kaliumcitrat, Kaliumacetat, Kaliumnitrat, Strontiumchlorid, Strontiumnitrat, Strontiumcitrat, Strontiumacetat und Strontiumlactat und Eugenol.

Das Eugenol kann mit Aromaölen gemischt in den Mund- und Zahnpflegemitteln enthalten sein. Bevorzugt ist es in Form des Nelkenknospenöls in den Zusammensetzungen enthalten.
Vorzugsweise enthalten die erfindungsgemäßen Mund- und Zahnpflegemittel mindestens 0,5 Gew.-% Kalium- oder Strontiumionen in der Form eines gelösten Salzes und mindestens 0,01 Gew.-% Eugenol in reiner Form oder in der des Nelkenknospenöls.

### Geschmacksstoffe

Vorzugsweise enthalten die erfindungsgemäßen Mittel Geschmacksstoffe, zu denen z. B. Süßungsmittel und/oder Aromaöle gehören.

Als Süßungsmittel eignen sich beispielsweise Saccharinate (insbesondere Natriumsaccharinat), Cyclamate (insbesondere Natriumcyclamat) sowie Sucrose, Lactose, Maltose oder Fructose.

Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle (Mischung) als auch in Form der hieraus isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine/mehrere daraus isolierte bzw. synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Schließlich können weitere übliche Hilfsmittel zur Verbesserung der Stabilität und der sensorischen Eigenschaften der Mund- und Zahnpflegemittel enthalten sein. Solche Hilfsmittel sind beispielsweise
- Vitamine, z. B. Retinol, Biotin, Tocopherol, Ascorbinsäure und deren Derivate (z. B. Ester, Salze);
- Pigmente, z. B. Titandioxid oder Zinkoxid;
- Gefärbte Pigmentpartikel, beispielsweise gefärbte Kieselsäurepartikel, wie sie z. B. unter der Verkaufsbezeichnung Sorbosil^{®}BFG 51, BFG 52 und BFG 53 oder Sorbosil^{®}2352 im Handel sind. Es können auch Gemische unterschiedlich gefärbter Pigmentpartikel verwendet werden. Solche, z. B. kräftig orange, rot oder blau gefärbten Gelkieselsäure-Partikel können in Mengen von 0,1 - 1,0 Gew.-% in den erfindungsgemäßen Mitteln enthalten sein;
- Bleichmittel wie beispielsweise Wasserstoffperoxid und Wasserstoffperoxid-Vorstufen;
- Farbstoffe;
- pH-Stellmittel und Puffersubstanzen, z. B. Natriumcitrat, Natriumbicarbonat oder Kalium- und Natriumphosphate,
- Konservierungsmittel, z.B. p-Hydroxybenzoesäuremethyl-, ethyl- oder -propylester, Natriumsorbat, Natriumbenzoat, Bromchlorophen oder Triclosan;
- wundheilende und entzündungshemmende Stoffe wie z. B. Allantoin, Harnstoff, Panthenol, Azulen oder Kamillenextrakt, Acetylsalicylsäure-Derivate, Alkali-Rhodanide;
- Mineralsalze wie Zink-, Magnesium- und Mangansalze, beispielsweise -sulfate.

Alle fakultativen Zahnpasteninhaltsstoffe sind zusammen in einer Menge von etwa 2 bis 10 Gew.-%, bezogen auf das Gesamtgewicht, in den erfindungsgemäßen Mitteln enthalten.

Ein zweiter Gegenstand der Erfindung ist die Verwendung des erfindungsgemäßen Mund- und Zahnreinigungsmittels zur Herstellung eines Kosmetikums zur Vorbeugung und Bekämpfung hypersensibler Zähne.

Unter Verwendung zur Herstellung eines Kosmetikums wird die nicht-therapeutische Verwendung des erfindungsgemäßen Mund- und Zahnreinigungsmittels zur täglichen Reinigung und Pflege der Zähne und des Mundraums verstanden.

Ein dritter Gegenstand der Erfindung ist die Verwendung des erfindungsgemäßen Mund- und Zahnpflegemittels zur Herstellung eines Kosmetikums zur Aufhellung der Zähne und zur Vorbeugung von Verfärbungen und Wiederverfärbungen.

Die Aufhellung der Zähne wird durch Vergleich von Zähnen, die mit dem erfindungsgemäßen Zahnreinigungsmittel behandelt wurden und Zähnen, die mit einer Vergleichsrezeptur behandelt wurden, mittels eines handelsüblichen Kolorimeters (Fa. Lange) bestimmt.

Ein vierter Gegenstand der Erfindung ist die Verwendung des erfindungsgemäßen Mund- und Zahnpflegemittels zur Herstellung eines Kosmetikums zur Vorbeugung gegen Plaqueadhäsion und der damit verbundenen Reduktion der Plaqueneubildung auf Zahnoberflächen. Dadurch kann die durch Fehlstellen geförderte Anhaftung von Plaque auf der Zahnoberfläche reduziert werden.

Ein fünfter Gegenstand der Erfindung ist die Verwendung des efindungsgemäßen Mund- und Zahnpflegemittels zur Herstellung eines Kosmetikums zur Remineralisation von Dentalläsionen.

Die Wirkung des Kompositmaterials kann durch die Kombination mit einer geeigneten Zahnbürste gefördert werden. Durch Wahl einer geeigneten Beborstung und eines geeigneten Designs der Bürste, insbesondere des Bürstenkopfes und des Schnittes, ist die Reinigungsleistung eines komposithaltigen Zahnpflegemittels bei hoher Deponierung möglich.

Als Verpackung der Kompositmaterialien enthaltenden, erfindungsgemäßen Zahnpflegezubereitungen kommen Tuben und Spender, Pumpen und andere die Dosierung erleichternde Verpackungen in Frage.

Die folgenden Beispiele sollen den Gegenstand der Erfindung näher erläutern, ohne ihn darauf zu beschränken.

Die verwendeten Mengenangaben beziehen sich, sofern nicht anders angegeben, auf Gew.-%.

### Beispiele

### 1) Beispiel für eine semiviskose Zahncremezubereitung

| | |
|---|---|
| **Neosorb^{®} 70/70¹** | **19,000** |
| **Glycerin 86%ig** | **18,000** |
| **Poliertonerde^{®} P10 feinst²** | **0,500** |
| **Sident^{®} 8³** | **14,000** |
| **zacycloheptan-2,2-DiPhosphonsäure** | **0,500** |
| **NaOH** | **0,158** |
| **Natrium-Saccharinat** | **0,200** |
| **Natrium-Fluorid** | **0,320** |
| **PHB Methylester** | **0,100** |
| **Di Natriumphosphat** | **0,100** |
| **Tri Natriumphosphat** | **0,050** |
| **Nanit in glycerischer Dispersion⁴** | **0,855** |
| **Keltrol^{®}F⁵** | **1,250** |
| **1,2 Propylenglykol** | **5,000** |
| **Tego Betain BL 215⁶** | **0,600** |
| **Texapon^{®} K12G⁷** | **1,500** |
| **Aroma** | **1,000** |
| **Wasser** | **ad 100** |

### 2) Beispiel für eine dünnflüssige Zahncremezubereitung

| | |
|---|---|
| **Neosorb^{®} 70/70** | **55,000** |
| **Sident^{®} 8** | **12,000** |
| **Pollertonerde^{®} P10** | **0,500** |
| **Di Natriumphosphat** | **0,200** |
| **Natrium-Fluorid** | **0,320** |
| **Natrium-Saccharinat** | **0,200** |
| **Natrium-Benzoat** | **0,490** |
| **Zinksulfat-7H20** | **0,088** |
| **Titandioxid** | **1,000** |
| **Nanit in glycerischer Dispersion** | **0,855** |
| **Polywachs^{®} 1550** | **1,000** |
| **Keltrol^{®} F** | **0,500** |
| **Texapon^{®} K 1296⁸** | **1,500** |
| **Tagat^{®}S⁹** | **0,500** |
| **Tego Betain BL 215** | **0,600** |
| **NaOH** | **0,316** |
| **Ethanol** | **2,000** |
| **Aroma** | **1,200** |
| **Wasser** | **ad 100** |

### 2) Beispiel für eine viskose (feste) Zahncreme

| | |
|---|---|
| **Neosorb^{®} 70/70** | **30,000** |
| **Poliertonerde P10** | **1,000** |
| **Phosphorsäure 85%ig** | **0,050** |
| **Sident^{®} 8** | **12,000** |
| **Sident^{®} 22 S¹⁰** | **8,000** |
| **Sipernat^{®} (FK) 320DS¹¹** | **1,000** |
| **Titandioxid** | **1,000** |
| **Nanit in glycerischer Dispersion** | **0,855** |
| **Di Natriumphosphat** | **0,200** |
| **Natrium-Fluorid** | **0,320** |
| **Natrium-Saccharinat** | **0,200** |
| **NaOH** | **0,158** |
| **Polydiol^{®} 400¹²** | **3,000** |
| **Cekol^{®} 2000 H¹³** | **1,000** |
| **Texapon^{®} K12 G** | **1,350** |
| **Aroma** | **1,000** |
| **Wasser** | **ad 100** |

Es wurde in Versuchen nachgewiesen, dass selbst bei Verwendung von Poliermitteln wie Aluminiumhydroxiden die Deponierwirkung erhalten bleibt. Diese Versuche wurden z.B. mit Dentinproben durchgeführt und das Aufwachsen von Hydroxylapatit beurteilt.

Es wurden die folgenden Handelsprodukte verwendet:
- 1: Neosorb^{®}: INCI: Sorbitol; ca. 70% in Wasser;
Hersteller: Roquette
- 2: Poliertonerde P10 feinst: INCI: Alumina
Hersteller: Alcoa
- 3: Sident^{®} 8: INCI: Hydrated Silica;
Hersteller: Degussa
- 4: Nanit in glycerischer Dispersion: Kompositmaterial gemäß Erfindung als
10%ige Dispersion in Glycerin
- 5: Keltrol^{®}F: INCI: Xanthan Gum
Hersteller: CP Kelco
- 6: Tego Betain^{®} BL 215: INCI: Cocamidopropyl Betaine; AS: 30%
Hersteller: Goldschmidt
- 7: Texapon^{®}K12G: INCI: Sodium Lauryl Sulfate; AS. 95-99%
Hersteller: Cognis
- 8: Texapon^{®}K1296: Sodium Lauryl Sulfate; AS: 90%
Hersteller: Cognis
- 9: Tagat^{®}S: INCI: PEG-30 Glyceryl Stearate Hersteller: Tego Cosmetics
- 10: Sident^{®}22S: INCI: Hydrated Silica;
Hersteller: Degussa
- 11: Sipernat^{®} FK 320 DS: INCI: Hydrated Silica;
Hersteller: Degussa
- 12: Polydiol^{®} 400: INCI: Poly ethylene glycol; MG 380-420
Hersteller: Cognis
- 13: Cekol^{®}2000H: INCI: Cellulose Gum
Hersteller: Noviant

## Patentansprüche

1. Mund- und Zahnpflegemittel, enthaltend
a) ein Kompositmaterial, enthaltend
- in Wasser schwerlösliche Calciumsalze in Form von nanopartikulären Primärteilchen mit einer Länge von 5 bis 150 nm und einem Querschnitt von 2 bis 50 nm und
- Proteinkomponenten, ausgewählt aus Proteinen, Proteinhydrolysaten und Proteinhydrolysat-Derivaten, und
b) 10 bis 35% eines Putzmittelgemisches,
**dadurch gekennzeichnet, dass** das Putzmittelgemisch 0,01 bis 5 Gew.-% Aluminiumoxid-Poliermittel enthält.

2. Mund- und Zahnpflegemittel nach Anspruch 1, **dadurch gekennzeichnet, dass** es einen RDA-Wert im Bereich von 50 bis 170 aufweist.

3. Mund- und Zahnpflegemittel nach einem der Ansprüche 1 oder 2, **dadurch gekennzeichnet, dass** die schwerlöslichen Calciumsalze ausgewählt sind aus Fluorapatit und/oder Hydroxylapatit.

4. Mund- und Zahnpflegemittel nach einem der Ansprüche 1 oder 3, **dadurch gekennzeichnet, dass** die Proteinkomponente ausgewählt ist aus strukturbildenden Proteinen wie Kollagen, Gelatine, Keratin, Casein, Weizenprotein, Reisprotein, Sojaprotein, Mandelprotein und deren Hydrolysaten und Hydrolysat-Derivaten.

5. Mund- und Zahnpflegemittel nach einem der Ansprüche 1 bis 4, **dadurch gekennzeichnet, dass** der Gehalt des Kompositmaterials 0,01 bis 10 Gew.-%, bezogen auf das Gesamtgewicht des Mittels, beträgt.

6. Mund- und Zahnpflegemittel nach einem der Ansprüche 1 bis 5, **dadurch gekennzeichnet, dass** die nanopartikulären Primärteilchen stäbchenförmig sind.

7. Mund- und Zahnpflegemittel nach einem der Ansprüche 1 bis 6, **dadurch gekennzeichnet, dass** die nanopartikulären Primärteilchen stäbchenförmige Kristalle mit einem mittleren Länge-zu-Breite-Verhältnis von 3 bis ≤ 5, insbesondere ca. 4, bilden.

8. Mund- und Zahnpflegemittel nach einem der Ansprüche 1 bis 7, **dadurch gekennzeichnet, dass** das Putzmittelgemisch weitere Putzmittel, insbesondere Kieselsäuren, Aluminiumhydroxid, Silikate, Erdalkalimetallcarbonate und/oder- hydrogencarbonaten enthält.

9. Mund- und Zahnpflegemittel nach einem der Anspruche 1 bis 8, **dadurch gekennzeichnet, dass** es mindestens eine Remineralisationsförderungs-Komponente enthält.

10. Mund- und Zahnpflegemittel nach Anspruch 9, **dadurch gekennzeichnet, dass** die Remineralisationsförderungs-Komponente ausgewählt ist aus Magnesiumsalzen.

11. Mund- und Zahnpflegemittel nach einem der Ansprüche 1 bis 10, **dadurch gekennzeichnet, dass** es als weitere Zahnpasteninhaltsstoffe Tenside, Feuchthaltemittel, Bindemittel und Wirkstoffe gegen Zahn- und Zahnfleischerkrankungen enthält.

12. Mund- und Zahnpflegemittel nach einem der Ansprüche 1 bis 11, **dadurch gekennzeichnet, dass** die Wirkstoffe Antikaries-Wirkstoffe, antimikrobielle Stoffe, Zahnsteininhibitoren, Wirkstoffe gegen hypersensible Zähne und/oder Gemische dieser Stoffe sind.

13. Verwendung eines Mund- und Zahnpflegemittels nach einem der Ansprüche 1 bis 12 zur Herstellung eines Kosmetikums zur Vorbeugung und/oder Bekämpfung hypersensibler Zähne.

14. Verwendung eines Mund- und Zahnpflegemittels nach einem der Ansprüche 1 bis 12 zur Herstellung eines Kosmetikums zur Aufhellung der Zähne und/oder zur Vorbeugung und/oder von Verfärbungen und Wiederverfärbungen.

15. Verwendung eines Mund- und Zahnpflegemittels nach einem der Ansprüche 1 bis 12 zur Herstellung eines Kosmetikums zur Vorbeugung gegen Plaqueadhäsion auf der Zahnoberfläche.

16. Verwendung eines Mund- und Zahnpflegemittels nach einem der Ansprüche 1 bis 12 zur Herstellung eines Kosmetikums zur Remineralisation von Dentalläsionen.

## Claims

1. An oral and dental hygiene product comprising
a) a composite material comprising
- calcium salts which are slightly soluble in water, in the form of nanoparticulate primary particles having a length of from 5 to 150 nm and a cross section of from 2 to 50 nm and
- protein components selected from proteins, protein hydrolysates and protein hydrolysate derivatives, and
b) 10 to 35% by weight of a cleaning agent mixture,
**characterized in that** the cleaning agent mixture comprises from 0.01 to 5% by weight of aluminum oxide polishing agent.

2. The oral and dental hygiene product as claimed in claim 1, **characterized in that** it has an RDA in the range from 50 to 170.

3. The oral and dental hygiene product as claimed in either of claims 1 or 2, **characterized in that** the slightly soluble calcium salts are selected from fluoroapatite and/or hydroxyapatite.

4. The oral and dental hygiene product as claimed in either of claims 1 or 3, **characterized in that** the protein component is selected from structure-forming proteins such as collagen, gelatin, keratin, casein, wheat protein, rice protein, soybean protein, almond protein and their hydrolysates and hydrolysate derivatives.

5. The oral and dental hygiene product as claimed in any of claims 1 to 4, **characterized in that** the content of the composite material is from 0.01 to 10% by weight based on the total weight of the product.

6. The oral and dental hygiene product as claimed in any of claims 1 to 5, **characterized in that** the nanoparticulate primary particles are rod-like.

7. The oral and dental hygiene product as claimed in any of claims 1 to 6, **characterized in that** the nanoparticulate primary particles form rod-like crystals having an average length-to-breadth ratio of 3 to ≤ 5, in particular about 4.

8. The oral and dental hygiene product as claimed in any of claims 1 to 7, **characterized in that** the cleaning agent mixture comprises further cleaning agents, in particular silicas, aluminum hydroxide, silicates, alkaline earth metal carbonates and/or bicarbonates.

9. The oral and dental hygiene product as claimed in any of claims 1 to 8, **characterized in that** it comprises at least one remineralization-promoting component.

10. The oral and dental hygiene product as claimed in claim 9, **characterized in that** the remineralization-promoting component is selected from magnesium salts.

11. The oral and dental hygiene product as claimed in any of claims 1 to 10, **characterized in that** it comprises as further toothpaste ingredients surfactants, humectants, binders and active substances to counter dental and gingival disorders.

12. The oral and dental hygiene product as claimed in any of claims 1 to 11, **characterized in that** the active substances are anticaries active substances, antimicrobial substances, tartar inhibitors, active substances to counter hypersensitive teeth and/or mixtures of these substances.

13. The use of an oral and dental hygiene product as claimed in any of claims 1 to 12, for manufacturing of a cosmetic for the prevention and/or control of hypersensitive teeth.

14. The use of an oral and dental hygiene product as claimed in any of claims 1 to 12, for manufacturing of a cosmetic for brightening the teeth and/or for preventing and/or discolorations and rediscolorations.

15. The use of an oral and dental hygiene product as claimed in any of claims 1 to 12, for manufacturing of a cosmetic for preventing adhesion of plaque on the tooth surface.

16. The use of an oral and dental hygiene product as claimed in any of claims 1 to 12, for manufacturing of a cosmetic for remineralization of dental lesions.

## Revendications

1. Agent de soin buccal et dentaire, contenant
a) un matériau composite, contenant
- des sels de calcium difficilement solubles dans l'eau sous forme de particules primaires nanoparticulaires, présentant une longueur de 5 à 150 nm et une section de 2 à 50 nm et
- des composants protéiniques, choisis parmi les protéines, les hydrolysats protéiniques et les dérivés d'hydrolysats protéiniques, et
b) 10 à 35% d'un mélange d'agents nettoyants
**caractérisé en ce que** le mélange d'agents nettoyants contient 0,01 à 5% en poids d'agent de polissage de type oxyde d'aluminium.

2. Agent de soin buccal et dentaire selon la revendication 1, **caractérisé en ce qu'**il présente une valeur RDA dans la plage de 50 à 170.

3. Agent de soin buccal et dentaire selon l'une quelconque des revendications 1 ou 2, **caractérisé en ce que** les sels de calcium difficilement solubles sont choisis parmi la fluoroapatite et/ou l'hydroxylapatite.

4. Agent de soin buccal et dentaire selon l'une quelconque des revendications 1 ou 3, **caractérisé en ce que** le composant protéinique est choisi parmi les protéines structurantes, telles que le collagène, la gélatine, la kératine, la caséine, les protéines de blé, de riz, de soja, d'amande et leurs hydrolysats et dérivés d'hydrolysats.

5. Agent de soin buccal et dentaire selon l'une quelconque des revendications 1 à 4, **caractérisé en ce que** la teneur en matériau composite est de 0,01 à 10% en poids, par rapport au poids total de l'agent.

6. Agent de soin buccal et dentaire selon l'une quelconque des revendications 1 à 5, **caractérisé en ce que** les particules primaires nanoparticulaires sont en forme de bâtons.

7. Agent de soin buccal et dentaire selon l'une quelconque des revendications 1 à 6, **caractérisé en ce que** les particules primaires nanoparticulaires forment des cristaux en forme de bâtons avec un rapport longueur à largeur moyen de 3 à ≤ 5, en particulier d'environ 4.

8. Agent de soin buccal et dentaire selon l'une quelconque des revendications 1 à 7, **caractérisé en ce que** le mélange d'agents à nettoyants contient d'autres agents à polir, en particulier des silices, de l'hydroxyde d'aluminium, des silicates, des carbonates de métal alcalino-terreux et/ou des hydrogénocarbonates de métal alcalino-terreux.

9. Agent de soin buccal et dentaire selon l'une quelconque des revendications 1 à 8, **caractérisé en ce qu'**il contient au moins un composant favorisant la reminéralisation.

10. Agent de soin buccal et dentaire selon la revendication 9, **caractérisé en ce que** le composant favorisant la reminéralisation est choisi parmi les sels de magnésium.

11. Agent de soin buccal et dentaire selon l'une quelconque des revendications 1 à 10, **caractérisé en ce qu'**il contient comme autres constituants de pâtes dentaires des agents tensioactifs, des agents de rétention de l'humidité, des liants et des substances actives contre les maladies des dents et des gencives.

12. Agent de soin buccal et dentaire selon l'une quelconque des revendications 1 à 11, **caractérisé en ce que** les substances actives sont des substances actives anticaries, des substances antimicrobiennes, des inhibiteurs de tartre, des substances actives contre les dents hypersensibles et/ou des mélanges de ces substances.

13. Utilisation d'un agent de soin buccal et dentaire selon l'une quelconque des revendications 1 à 12 pour la préparation d'un produit cosmétique destiné à prévenir et/ou à lutter contre les dents hypersensibles.

14. Utilisation d'un agent de soin buccal et dentaire selon l'une quelconque des revendications 1 à 12 pour la préparation d'un produit cosmétique destiné à éclaircir les dents et/ou à prévenir les colorations et les recolorations.

15. Utilisation d'un agent de soin buccal et dentaire selon l'une quelconque des revendications 1 à 12 pour la préparation d'un produit cosmétique destiné à la prévention de l'adhérence de la plaque à la surface des dents.

16. Utilisation d'un agent de soin buccal et dentaire selon l'une quelconque des revendications 1 à 12 pour la préparation d'un produit cosmétique pour la reminéralisation de lésions dentaires.
